Europäisches Patentamt

European Patent Office

Office européen des brevets

(19)

(11) **EP 1 156 802 B1**

(12) **EUROPEAN PATENT SPECIFICATION**

(45) Date of publication and mention
of the grant of the patent:
**07.12.2005   Bulletin 2005/49**

(51) Int Cl.[7]: **A61K 31/385**
// A61K31:385, A61K31:195

(21) Application number: **00907644.9**

(22) Date of filing: **28.02.2000**

(86) International application number:
**PCT/EP2000/001637**

(87) International publication number:
**WO 2000/053176 (14.09.2000 Gazette 2000/37)**

(54) **PHARMACEUTIC, DIETETIC AND COSMETIC COMPOSITIONS BASED ON THIOCTIC ACID AND CYSTEINE**

KOSMETISCHE, DIÄTETISCHE ODER PHARMAZEUTISCHE ZUSAMMENSETZUNG DIE THIOCTSÄURE UND CYSTEIN ENTHALTEN

COMPOSITIONS PHARMACEUTIQUES, DIETETIQUES ET COSMETIQUES A BASE D'ACIDE ALPHA-LIPOIQUE ET DE CYSTEINE

(84) Designated Contracting States:
**AT BE CH CY DE DK ES FI FR GB GR IE IT LI LU MC NL PT SE**

(30) Priority:  **05.03.1999  IT  MI990460**

(43) Date of publication of application:
**28.11.2001   Bulletin 2001/48**

(73) Proprietor: **Uni-CI S.r.l**
**20125 Milano (IT)**

(72) Inventors:
 • **DALL'AGLIO, Roberto**
  **I-43100 Parma (IT)**
 • **BORGONOVO, Margherita**
  **I-20154 Milano (IT)**

 • **INTROINI, Carlo**
  **I-20149 Milano (IT)**
 • **MELEGARI, Pierangelo**
  **I-43027 San Prospero (IT)**

(74) Representative: **Mancini, Vincenzo et al**
**Giambrocono & C. s.p.a.,**
**Via Rosolino Pilo 19/B**
**20129 Milano (IT)**

(56) References cited:
 **WO-A-98/30228**          **WO-A-98/33494**
 **DE-A- 4 328 871**         **FR-M- 4 630**
 **US-A- 4 990 330**         **US-A- 5 292 538**
 **US-A- 5 904 924**

**EP 1 156 802 B1**

**Description**

The present invention concerns, novel pharmaceutic, dietetic and cosmetic compositions useful for the prevention and treatment of conditions caused by oxidative stresses and alterations of mitochondrial energetic metabolism.

The compositions of the invention comprise in particular tioctic acid and cysteine and/or a pharmaceutically, dietetically or cosmetically acceptable derivative thereof.

Tioctic acid, known also as $\alpha$-lipoic acid, is employed in the treatment of hepatic pathologies and as an antidote for poisonous mushrooms of Amanita species genus.

It is known that tioctic acid, owing to its structure, is both watersoluble and liposoluble in nature and has therefore the capability of acting equally in intracellular and extracellular environment and reducing both the endogenous and exogenous free-radicals.

Cysteine is a semi-essential amino acid, component of glutathione, cystine and many other proteins; it is stable in acid environment and is employed as a detoxicating agent.

It is further known that cysteine, as well as the pharmaceutically, dietetically or cosmetically acceptable derivatives thereof, are antioxidants which possess the same activity of reduction of free-radicals as tioctic acid.

It is also known that association of tioctic acid and cysteine and/or a pharmaceutically, dietetically or cosmetically acceptable derivative thereof, is advantageous for the prevention and treatment of a condition caused by oxidative stresses.

In the present specification, the expression "oxidative stress" means any physiological and/or pathological condition characterised by an increase of the production of peroxides and free-radicals in general, for example in conditions of physical or mental stress, protracted fatigue, acute viral disease, hepatic insufficiency, dysmetabolism, especially referred to diabetes, inflammatory processes, both acute and chronic, such as, for instance, asthma, rheumatic and rheumatoid arthritis, Crohn's disease and ulcerative colitis, which always bring about a free-radical damage beyond the levels useful for defensive purposes and involving also the interested tissues, and ageing as a consequence also of oxidative phenomena, etc.

The association acts also specifically for optimising the lipidic and carbohydrate metabolisms and reducing the intra- and extra-mitochondrial oxidative metabolism, which is the main source of free-radicals.

In a composition containing tioctic acid and cysteine and/or a pharmaceutically, dietetically or cosmetically acceptable derivative thereof, the concentration of each component may amount from 0,1 to 40,0% by weight, preferably between 0,1 and 20,0% by weight, in addition to an acceptable vehicle and/or excipient.

Among the pharmaceutically, dietetically or cosmetically acceptable derivative of cysteine, preferred are N-acetylcysteine, cystine and carboxymethyl cysteine, although any derivative could be selected by the skilled in the art.

The WO 98/33494 A and the US-A-5 292 538 disclose compositions in which the weight ratio of the thioctic acid is much lower than that of the antioxidants present in the same compositions.

According to the present invention, a clear synergic effect deriving from the association of the above-mentioned molecules has been in fact observed when the weight ratio of the thioctic acid over the antioxidant is of about 1:1 w/w and it can be supposed that an increase of the activity of the individual components of the composition of the invention on glutathione peroxidase is the cause of such a surprising result. A further synergic effect improves the efficiency of mitochondrial oxidative metabolism, consequently reducing the free-radicals and improving the treatment of diseases deriving from "oxidative stresses".

It has now been found that the compositions of the invention are useful for preparing a drug, a cosmetic or a dietetic supplement for the prevention and/or treatment of physiological and/or pathological conditions caused by oxidative stresses involving, therefore, an increase of production of free-radicals (peroxides, etc.)

The composition of the invention is therefore useful for the prevention and/or treatment of physical and/or mental stress, pain and asthenia, the Down's syndrome, chronic degenerative pathologies, such as Alzheimer's disease, acute and chronic viral diseases, hepatic insufficiency, dysmetabolism, particularly in the clinical conditions of decreased tolerance to glucose characterised by hyperinsulinism and weight increase, diabetes, obesity, membranopathies as liposclerosis and cellulitis; cardiopathies, particularly those characterised by vasospasms, for example angina pectoris, myocardium infarction, for the protection of coronary vessels, prevention and/or treatment of ischemia.

The compositions of the invention are further useful for the prevention and/or treatment of viral pathologies, particularly herpes, influenza and B and C hepatitis.

It has been observed a clear improvement of patients affected by immunopathologies, particularly the ones involving immunoregulation and the acquired immuno-deficiency syndrome (AIDS).

The composition of the invention proved to be surprisingly useful in the treatment of climacteric and premenstrual syndromes and during pregnancy which is a physiological state accompanied by a high metabolic rate and consequently elevated production of reactive oxygen species (ROS).

Particularly, pre-eclampsia, a pregnancy-specific condition characterised by hypertension and proteinuria both of which remit after delivery, is associated with increased lipid peroxidation in the maternal circulation and in placenta compared with the ones measured during normal pregnancy. Even the humour tone (depression), which is a side, but very frequent, symptom of menopause and after delivery, has been improved by the administration of the compositions of the invention, which show clearly their usefulness also in the treatment of depression or anxiety conditions.

Further, the composition of the invention showed to be useful for the preparation of a medicament for the prevention and the treatment of sperm motility: the production of sperm-generated ROS is accelerated in defective sperm and it highly correlates with impairment in sperm motility. The ability of the composition of the invention to hinder the formation of free radicals resulted to be useful also for increasing the fertilisation rate in asthenospermic people.

Still further, the compositions of the invention proved to be useful for the prevention and/or the treatment of malignant and benign tumors, particularly of cheloids.

Also the prevention and/or the treatment of retinitis pigmentosa, cataract and age related macular degeneration proved to be possible using the compositions of the invention.

It has been still further observed an increased fatigue resistance, a protein saving power and a protection of muscles under stress from the oxidative damages.

From the experimental data of tests carried out on athletes, a significant decrease of fat mass has been observed, even in anaerobiosis conditions. The lipid reduction is higher in the sites where the lipid content is higher, which suggests an optimal normalisation of oxidative metabolism of lipids.

The anti-free-radicals activity of the compositions of the invention makes them suitable also for the preparation of topical, pharmaceutic and cosmetic formulations (creams, ointments, salves, etc.) and of a dietetic supplement for preventing and treating general atopy, atopical dermatitis, psoriasis, acne, bedsores, solar erythemas, and for hindering the process of ageing, particularly skin ageing, hair loss, the damages induced by smoke and for the protection of muscles stress from oxidative damages.

The compositions of the invention are also useful for the preparation of a drug for the prevention and treatment of phlebopathies, such as ulcers, and inflammatory phenomena, conditions wherein the presence of free-radicals is usually high, an analgesic effect being also observed.

The surprising synergic effect shown by the association of tioctic acid and cysteine and/or a pharmaceutically, dietetically or cosmetically acceptable derivative thereof when used together in the weight ratio of about 1:1 w/w can be further increased by addition to the compositions of the invention of other anti-free-radical agents, such as coenzyme Q10, folic acid, carnitine (or a carnitine derivative which can be easily selected by the skilled in the art, such as the methyl-, propyl- carnitine, etc.), vitamins B, C and E, flavonoids, pantothenic acid, terpenes, tannins, natural extracts such as Tarchonanthus Canphoratus L (both as an essential oil and as an extract), oligo-elements, for example selenium, chromium, zinc, copper etc., polyphenols, resveratrol, anthocyanidins, or essential fatty acids, for example the omega-3-ones (for example, by employing oils with a high content of such acid, such as linseed oil, perilla oil, etc.), phytoestrogens (for example those extracted from soybeans) and aminoacids (glutamine, glutamic and aspartic acids, etc.), each one in amounts between 2 and 8% by weight.

The compositions of the invention can further comprise, preferably, melatonine, alone or conjugated with adenosine, or one of its derivatives, such as 2-bromomelatonine, and/or allantoine, each one in amounts of 0.5-5.0% by weight.

It is also preferable adding to the compositions of the invention a medium-chain ($C_6$-$C_{12}$) triglyceride, in amounts of 5-40% by weight.

The compositions of the invention can be obviously formulated, depending on the selected realisation form, administration route and specific purposes, by employing the usual pharmaceutic, dietetic and cosmetic techniques known by those skilled in the field.

The compositions of the invention can be provided in the most different forms, according to their use and administration route, the subject to be treated, concentration etc., on the basis of the knowledge of those skilled in the field. Particularly preferred are tablets and capsules, but also powders, granules to be dispersed in water, pills, even the effervescent ones, syrups, confectioneries, topical and injectable preparations, plasters for transdermal release, suppositories or solutions, emulsions and/or dispersions for aerosol.

Also the vehicles and/or excipients comprised in the compositions of the invention can be selected, according to the use, administration form etc., by those skilled in the field on the basis of their general knowledge.

For example, vehicles for the compositions of the invention can be liposomes, nanospheres, acrylic foams, cyclodextrins, etc., excipients can be magnesium hydroxide, magnesium oxide, vegetable extracts (such as, for example, soybean flour, wheat flour, wheat and soybean proteins, etc.), polysaccharides (such as, for example, cellulose, starches, etc.), polyalcohols (such as, for example, glycerol, sorbitol, mannitol, polyethylene glycol, etc.), lipids (such as, for example, sunflower oil, olive oil, karitè butter, alkyl esters of polyacids, alkyl maleate, alkyl tartrate), emulsifiers (such as, for example, ethoxylate alcohols, soaps, acyl glutamates), polymers as viscosity improving agents (such as, for example, acrylic resins, modified cellulose, guar gum, etc.), preservatives (such as, for example, imidazolidinylurea, phenoxyethanol, parahydroxybenzoates, metallic silver, etc.), fragrances (such as, for example, compositions of essential, natural and synthetic, oils), coloring agents or pigments (such as, for example, carmine blue, tartrazine, titanium dioxide, zinc oxide, etc.), sweeteners (such as, for example, saccarose, sorbitol, aspartame, etc.).

The following examples illustrate the invention without limiting it.

EXAMPLE 1

Muscles under stress

N-acetylcysteine (200 mg) and magnesium hydroxide (150 mg) are mixed and powdered in a mortar and then tioctic acid (200 mg) is added until an homogeneous mixture is obtained. With the above obtained mixture, type O gelatine capsules (produced by CAPSUGEL PARK DAVIS) are filled.

The preferred composition was orally administered [tioctic acid (10 mg/Kg/day) and N-acetylcysteine (10 mg/Kg/day)] to twelve athletes of body building, for a period of 45 or 60 days, and weight and fat percent were controlled by plicometry.

The values of athletes with 60 days of treatment show a decrease of weight (4%) and a sharp decrease of fat percentage (7%). There is an improvement of proteic mass of the muscle of 3%.

As far as data of the group whose treatment was interrupted after 45 days are concerned, a decreasing trend, even if less sharp, is observed also after the interruption.

| Athletes with supplement for 60 days | | | | | | |
|---|---|---|---|---|---|---|
| ATHLETES | AGE 28-44 | INITIAL WEIGHT KG | INITIAL FAT % | 45 DAYS FAT % | 60 DAYS WEIGHT KG | 60 DAYS FAT % |
| 1)MA | 34 | 84.8 | 12.6 | 6 | 81.1 | 4.1 |
| 3)CR | 34 | 82.9 | 9.4 | 5.4 | 80.2 | 4.0 |
| 4)GF | 32 | 88.5 | 10.6 | 6.3 | 86.5 | 3.6 |
| 5)BR | 44 | 94.7 | 9.5 | 6.1 | 92.5 | 3.5 |
| 8)RR | 29 | 86.7 | 14.1 | 7.8 | 84.0 | 4.9 |
| 9)RM | 40 | 114.5 | 14.4 | 7.5 | 103.3 | 4.8 |
| 11)MG | 38 | 102.4 | 10.6 | 6.7 | 100.4 | 4.9 |
| 12)RA | 40 | 98.6 | 8.9 | 5.6 | 97.0 | 3.4 |
| AVERAGE | 36.4 | 94.1 | 11.3 | 6.4 | 90.6 | 4.2 |
| SIGMA | 5.0 | 10.7 | 2.2 | 0.9 | 8.9 | 0.6 |
| *Prob=66%* | | | | | | |
| Lower Threshold | | 83.4 | 9.1 | 5.6 | 81.7 | 3.5 |
| Higher Threshold | | 104.9 | 13.4 | 7.3 | 99.6 | 4.8 |
| Width | | 21.4 | 4.3 | 1.7 | 17.9 | 1.3 |

| Athletes with supplement for 45 days | | | | | | |
|---|---|---|---|---|---|---|
| ATHLETES | AGE 28-44 | INITIAL WEIGHT KG | INITIAL FAT % | 45 DAYS FAT % | 60 DAYS WEIGHT KG | 60 DAYS FAT % |
| 2) LL | 28 | 86.4 | 14.3 | 6.5 | 81.3 | 5.5 |
| 6) SG | 31 | 79.4 | 10.8 | 6.4 | 74.3 | 5.2 |
| 7) RC | 33 | 88.5 | 11.4 | 6.3 | 81.2 | 5.7 |
| 10) PM | 31 | 96.8 | 14.8 | 6.8 | 88.3 | 5.7 |

(continued)

| Athletes with supplement for 45 days | | | | | | |
|---|---|---|---|---|---|---|
| ATHLETES | AGE 28-44 | INITIAL WEIGHT KG | INITIAL FAT % | 45 DAYS FAT % | 60 DAYS WEIGHT KG | 60 DAYS FAT % |
| AVERAGE | 30.8 | 87.8 | 12.8 | 6.5 | 81.3 | 5.5 |
| SIGMA | 2.1 | 7.2 | 2.0 | 0.2 | 5.7 | 0.2 |
| *Prob=66%* | | | | | | |
| Lower Threshold | | 80.6 | 10.8 | 6.3 | 75.6 | 5.3 |
| Higher Threshold | | 94.9 | 14.8 | 6.7 | 87.0 | 5.8 |
| Width | | 14.3 | 4.0 | 0.4 | 11.4 | 0.5 |

The statistical analysis of data shows that the percent reduction of fat tends to a lower dispersion (standard deviation or width). The composition of the invention seems therefore tending to regulate the fat percent in the muscles to a constant value.

The fat reduction effect is more evident clear where the percentage is more shifted from the ideal physiologic value. All the athletes pointed out a minor fatigue and a better resistance during the training.

EXAMPLE 2

Hepatic insufficiency

The composition of Example 1 was orally administered [tioctic acid (10 mg/Kg/day) and N-acetylcysteine 10 mg/kg/day)] to twelve patients and the hepatic functionality was then evaluated.

A) values determined before the administration of the composition of the invention.
B) values determined 45 days after the administration (3 patients spontaneously interrupted the administration of the composition)
GPT = glutamic - pyruvic transaminase
GOT = glutamic - oxalacetic transaminase
TG = triglycerides

| Patient | MA | | LL | | CR | | GF | |
|---|---|---|---|---|---|---|---|---|
| | A | B | A | B | A | B | A | B |
| GPT | 60 | 23 | 96 | 40 | 78 | 41 | 41 | 31 |
| GOT | 42 | 18 | 65 | 22 | 53 | 34 | 36 | 18 |
| TG | 260 | 192 | 190 | 135 | 280 | 147 | 150 | 102 |
| Patient | BR | | SG | | RC | | RR | |
| | A | B | A | B | A | B | A | B |
| GPT | 115 | 54 | 56 | 41 | 91 | - | 32 | 28 |
| GOT | 92 | 91 | 52 | 28 | 75 | | 22 | 20 |
| TG | 361 | 163 | 214 | 115 | 272 | | 169 | 116 |
| Patient | RM | | PM | | MG | | RA | |
| | A | B | A | B | A | B | A | B |
| GPT | 124 | 41 | 42 | - | 102 | 64 | 88 | - |

(continued)

| Patient | RM | | PM | | MG | | RA | |
|---|---|---|---|---|---|---|---|---|
| | A | B | A | B | A | B | A | B |
| GOT | 92 | 28 | 38 | - | 84 | 41 | 91 | - |
| TG | 301 | 110 | 154 | | 209 | 131 | 284 | - |

The table below reports the values of the transaminases measured before and 45 days after the administration of the composition.

| PATIENT | GPT | GPT45 | GOT | GOT45 | TG | TG45 |
|---|---|---|---|---|---|---|
| | | | | | | |
| MA | 60 | 23 | 42 | 18 | 260 | 192 |
| II | 96 | 40 | 65 | 22 | 190 | 135 |
| CR | 78 | 41 | 53 | 34 | 280 | 147 |
| GF | 41 | 31 | 36 | 18 | 150 | 102 |
| BR | 115 | 94 | 92 | 91 | 361 | 163 |
| SG | 56 | 41 | 52 | 28 | 214 | 115 |
| RR | 32 | 28 | 22 | 20 . | 169 | 116 |
| RM | 124 | 41 | 92 | 28 | 301 | 110 |
| MG | 102 | 64 | 84 | 41 | 209 | 131 |
| AVERAGES | 78.2 | 44.7 | 59.7 | 33.3 | 237.1 | 134.6 |
| SIGMA | 32.9 | 21.8 | 25.3 | 22.9 | 68.5 | 28.9 |

The data analysis allows a clear improvement of hepatic metabolism to be observed.

The reduction of the free-radicals obtained by the administration of the composition of the invention has therefore brought about a hepatoprotective activity with the reduction of transaminases. The reduction of the triglycerides is supposed to be dependent on a better mitrochondrial utilisation.

EXAMPLE 3

Menopause/humour tone

The composition of Example 1 [tioctic acid (200 mg/Kg/day) and N-acetylcysteine (200 mg/Kg/day)] was orally administered to five women 42-51 years old, for three months.

Further, the psychological conditions of the five women were evaluated according to the Hamilton method by determining the levels of the luteo-plasmatic hormone and the "social satisfaction"

| Luteo-plasmatic hormone (U.I./g) | | |
|---|---|---|
| SUBJECTS | INITIAL | AFTER 3 MONTHS |
| PL | 61 | 51 |
| VT | 49 | 45 |
| DT | 50 | 44 |
| FS | 29 | 41 |
| CE | 75 | 50 |
| AVERAGE | 52.8 | 46.2 |
| SD | 18.84 | 3.74 |

EP 1 156 802 B1

HAMILTON TEST

Depression scale according to Hamilton

| EUPHORIA | < 30 |
|---|---|
| NORMAL CONDITION | 30-35 |
| DEPRESSION | > 35 |

| SUBJECTS | INITIAL | AFTER 3 MONTHS |
|---|---|---|
| PL | 56 | 36 |
| VT | 56 | 41 |
| DT | 43 | 31 |
| FS | 51 | 36 |
| CE | 49 | 34 |
| AVERAGE | 51 | 35.6 |
| SD | 5.38 | 4.20 |

"Social satisfaction": self-evaluation scale to social adaptation - Normal values: 35-52

| PL | 27 | 39 |
|---|---|---|
| VT | 31 | 41 |
| DT | 25 | 46 |
| FS | 20 | 35 |
| CE | 32 | 38 |
| AVERAGE | 27 | 40 |
| SD | 5.60 | 4.69 |

All the subjects who received the treatment showed a sharp improvement of the humour and even more of the "social satisfaction".
The administration of the composition of the invention has brought about a sharp improvement for the subjects in menopause.
The reduction of the luteo hormone further shows a decrease of fatty tissues.

EXAMPLE 4

Viral pathologies

The number of infections and their seriousness were evaluated in six healthy children between 4 and 6 years old by oral administration of the composition obtained by mixing and powdering in a mortar:
N-acetylcysteine (50 mg) and magnesium hydroxide (30 mg) were admixed then adding tioctic acid (50 mg) until an homogeneous mixture was obtained. With this mixture, gelatine capsules type O (produced by Capsugel Park Davis) were filled. An analogous, but non-treated, group of children, was utilised as control.
The evaluation, for more than three months, was carried out when all the children, even those of the control group, attended the nursery school.
The number of infectious events of viral nature, treated with symptomatic drugs, their average duration and the arising of complications (otitis, sinusitis, tonsillitis, bronchitis, for which the antibiotic therapy was necessary), were determined.

| | age | N. of infective events | average duration | complications |
|---|---|---|---|---|
| AL | 3 | 3 | 3 days | 2 |
| CM | 4 | 2 | 2.5 | 1 |
| BE | 4.5 | 1 | 3 | 0 |

(continued)

|  | age | N. of infective events | average duration | complications |
|---|---|---|---|---|
| MM | 4.5 | 2 | 2 | 0 |
| BA | 5 | 1 | 2 | 0 |
| BE | 6 | 1 | 2 | 0 |

| Control group |  |  |  |  |
|---|---|---|---|---|
|  | Age | N. of infective events | average duration | complications |
| IC | 3 | 4 | 5 days | 2 |
| BM | 4 | 3 | 5 | 2 |
| FF | 4.5 | 3 | 4 | 1 |
| AS | 4.5 | 3 | 4 | 1 |
| CA | 5 | 2 | 3 | 0 |
| BL | 6 | 2 | 3 | 0 |

The effectiveness of the composition of the invention for reducing the number of infections events and their seriousness has been therefore proved.

EXAMPLE 5

Dermatological pathologies

Six psoriasis patients were treated with the following emulsion "E1" daily applied and prepared as follows:

| PHASE A | % |
|---|---|
| Tioctic acid (produced by Antibioticos) | 0.5 |
| N-acetylcysteine (produced by Agar) | 0.5 |
| Ethanol alcohol (produced by Orbat) | 10 |
| Ceramide (produced by Quest) | 0.2 |
| Lecithin (produced by Rhone Poulenc) | 2.5 |
| Deionized water | 20 |
| PHASE B |  |
| Deionized water | a 100 |
| Dipotassium glycirrhate (produced by Nikkon) | 0.005 |
| Fucogel (polysaccharides by CFM) | 3 |
| Glycerol (produced by Henkel) | 1.5 |
| 1.3-butylen glycol (produced by LCM) | 1.5 |
| Imidazolidinyl urea | 0.25 |
| Phenoxy ethanol | 0.25 |
| Carbopol 2000 (produced by Biochim) | 0.2 |
| Pemulen TR-2 (produced by Biochim) | 0.5 |
| PHASE C |  |
| Alkyl maleate (produced by Condea) | 2 |
| Alkyl tartrate (produced by Condea) | 2 |
| Macadamia nut oil (produced by Balestrini) | 1 |
| Shorea butter (produced by Gianni) | 1 |

(continued)

| PHASE C | |
|---|---|
| Jojoba oil (produced by Balestrini) | 1 |
| PHASE D | |
| Triethanolamine (produced by Gianni) | 0.7 |
| PHASE E | |
| Fragrancies(CONC 40648 produced by CERIZA) | 0.2 |

Phase A: a dispersion of ethanol in lecithin and in the other components is preferred, water is then added and the mixture is emulsified for 10 minutes. Phase B: in a dispersion of Carbopol 2000 and Pemulen TR-Z in water at 70°C the other components are dissolved.

Phase C: the components are mixed by heating to 70°C and the phase C is dispersed into the phase B by emulsifying for 10 minutes; phase D is added by uniformly mixing and the mixture is cooled. At 40°C the phase A is added by continuously cooling under uniform mixing, the phase E is then added. Cooling is continued till 30°C.

With the same procedure of emulsion "E1", also the emulsion "E2", containing tioctic acid and N-acetylcysteine, both in amounts of 1,25% by weight, and emulsion "E3", containing tioctic acid and N-acetylcysteine, both in amounts of 2,5% by weight, were prepared.

In order to test more clearly the emulsions of the invention, the test was carried out in winter when psoriasis worsens.

| Effects on the skin | | | | | |
|---|---|---|---|---|---|
| Patient | Age | m/f | Treatment | Seriousness/Site | Results |
| MM | 42 | m | E1 for 15 days | +knee | regression |
| CF | 23 | m | E1 for 30 days | +elbow | regression |
| BML | 51 | f | E2 for 30 days | +++plantar | excellent |
| CA | 30 | m | E3 for 15 days | +elbow | good |
| VP | 35 | m | E3 for 20 days | ++limbs | good |
| CC | 75 | m | E3 for 20 days | ++limbs/knee | fair |

EXAMPLE 6

Phlebopathies

The composition of Example 1 [tioctic acid (200 mg/day) and N-acetyl cysteine (200 mg/day)] was added to the usual anti-coagulative therapy based on coumarin derivatives and administered to 4 women 50 and 65 years old, respectively, suffering from deep thrombophlebitis of the lower limbs, for 2 months. The signs of the venous stasis, clinically assessed by congestion, cyanosis and edema, showed a faster regression in comparison with other patients of the same pathology and in analogous conditions, treated with the coumarin-derivatives alone. The patients complained lower pain and discomfort and had less resort to anti-inflammatory and/or analgesic drugs.

The above composition of the invention was also administered to six patients, affected by chronic venous insufficiency, for 3 months but with a double daily dose [tioctic acid (400 mg/day) and N-acetylcysteine (400 mg/day)]. A sharp regression of some symptoms, such as, for example, heaviness of the legs, tingling and evening oedema, was observed just after 15 days of therapy.

For the evaluation of the results the following quantitative scale has been employed:

| 0 | no reduction |
|---|---|
| 1 | moderate reduction |
| 2 | middle reduction (>30%) |
| 3 | excellent reduction (>50%) |
| 4 | complete disappearance |

| PATIENT | AGE | LEG HEAVINESS | TINGLING | OEDEMA |
|---------|-----|---------------|----------|--------|
| AL | 45 | 2 | 3 | 2 |
| BM | 51 | 2 | 2 | 2 |
| DR | 48 | 3 | 3 | 2 |
| CD | 54 | 2 | 2 | 1 |
| BL | 47 | 1 | 1 | 0 |
| MM | 56 | 2 | 2 | 1 |

EXAMPLE 7

CLINICAL TEST ON VOLUNTEERS AFFECTED BY LIPOSCLEROSIS

A cream of the invention (composition VI), containing 2% tioctic acid / 2% cysteine was compared with a cream containing a placebo formula, using a double blind test.

A standard population of women 22-40 years old affected by liposclerosis (cellulitis) was separated in two groups in a random way. The parameters to be evaluated during the test were not significantly different as controlled using the Student's t test.

    1) GS Study Group: 15 women applied the cream of the invention
    2) GC Control Group: 16 women applied the placebo cream

All the volunteers applied the creams daily for a month and were submitted to physical standardized activity (watergym) for two hours a week in two sections.

The diet was free and it was controlled that the volunteers did not start any hormone therapy like oestrogen or slimming diet or important variation in the nourishment. The plicae on the thigh (QU = quadriceps plica, PT = kneecap plica, FE = femoral plica) and two circumferences (CR = root of thigh, CM = median thigh) were measured.

A statistical analysis of the results using the Student's t test was performed. The data did not show any difference between the two groups GS and GC before treatment. The measures regarding the thickness of plicae resulted lower in both groups, but only the data relating to group GS resulted to be statistically significative (p < 0,005).

The Student's t test was also performed between the two groups GS/GC as far as the percentual weight loss was concerned. The results were evaluated measuring the values of the plicae before (t0) and after (t1) the treatment, according to the following formula:

$$[(plica\ t0 - plica\ t1)/plica\ t0]x100$$

The results obtained, showed again to be statistically significative (QU = p<0.005, PT = p<0.05, FE = p=0.05) for each plica.

No significative decrease of the circumferences was noted after the treatment also because all volunteers were under controlled physical activity that have increased their muscular mass as already demonstrated on the body building test.

It is therefore possible to note that the cream of the invention reduces the subcutaneous fat and liposclerosis. The skin after treatment resulted smoother insofar as both appearance and touch are concerned; less nodules were noted too.

| CONTROL GROUP (GC) | | | | | | | | | |
|---|---|---|---|---|---|---|---|---|---|
| Code | QUO | QU1 | PTO | PT1 | FE0 | FE1 | CR0 | CR1 | CM0 | CM1 |
| | | | | | | | | | | |
| FS.01 | 30,0 | 31,0 | 13,8 | 13,8 | 21,0 | 21,0 | 60,0 | 60,0 | 53,0 | 53,5 |
| | | | | | | | | | | |
| FS.02 | 31,4 | 30,4 | 20,0 | 19,0 | 12,2 | 12,1 | 59,0 | 58,5 | 54,0 | 53,0 |

(continued)

| CONTROL GROUP (GC) | | | | | | | | | |
|---|---|---|---|---|---|---|---|---|---|
| Code | QUO | QU1 | PTO | PT1 | FE0 | FE1 | CR0 | CR1 | CM0 | CM1 |
| FS.03 | 21,0 | 19,8 | 6,8 | 4,6 | 9,4 | 9,4 | 54,5 | 54,5 | 51,0 | 51,0 |
| FS.04 | 24,2 | 23,2 | 12,2 | 12,0 | 32,0 | 30,0 | 60,0 | 60,0 | 53,0 | 53,0 |
| FS.05 | 23,0 | 22,0 | 12,2 | 12,2 | 19,0 | 18,6 | 53,5 | 53,8 | 47,3 | 49,0 |
| FS.06 | 26,0 | 25,8 | 16,2 | 16,2 | 33,0 | 32,0 | 63,0 | 62,5 | 57,5 | 55,0 |
| FS.07 | 13,2 | 13,2 | 11,2 | 11,2 | 10,4 | 10,5 | 52,6 | 52,6 | 47,3 | 47,2 |
| FS.08 | 37,2 | 37,2 | 26,0 | 26,0 | 21,8 | 22,0 | 57,0 | 57,0 | 50,5 | 51,0 |
| FS.09 | 19,0 | 18,6 | 16,4 | 15,8 | 14,2 | 14,0 | 59,0 | 58,8 | 54,5 | 54,2 |
| FS.10 | 12,0 | 12,0 | 9,0 | 8,6 | 16,2 | 15,5 | 52,0 | 51,8 | 49,6 | 48,8 |
| FS.11 | 39,0 | 39,0 | 23,6 | 24,0 | 24,0 | 63,4 | 63,4 | 57,5 | 57,5 | 57,5 |
| FS.12 | 23,2 | 22,6 | 16,4 | 16,0 | 28,6 | 28,2 | 55,0 | 54,5 | 49,2 | 49,8 |
| FS.13 | 29,4 | 28,8 | 19,2 | 18,5 | 26,0 | 24,8 | 57,0 | 56,0 | 52,4 | 51,5 |
| FS.14 | 15,0 | 14,2 | 7,0 | 6,8 | 6,2 | 5,8 | 54,5 | 54,0 | 50,0 | 49,4 |
| FS.15 | 17,3 | 17,3 | 12,2 | 12,0 | 18,4 | 18,0 | 57,5 | 57,0 | 52,5 | 52,0 |
| FS.16 | 33,2 | 32,8 | 17,8 | 17,2 | 17,6 | 17,0 | 56,6 | 56,0 | 52,8 | 52,2 |
| STUDY GROUP (GS) | | | | | | | | | |
| Code | QUO | QU1 | PT0 | PT1 | FE0 | FE1 | CR0 | CR1 | CM0 | CM1 |
| FS.01 | 31,6 | 29,4 | 17,8 | 1,8 | 23,4 | 19,6 | 60,5 | 59,0 | 52,8 | 51,4 |
| FS.02 | 27,0 | 27,4 | 13,6 | 13,2 | 23,0 | 23,3 | 58,0 | 58,0 | 49,5 | 49,5 |
| FS.03 | 29,4 | 26,4 | 19,0 | 16,0 | 19,0 | 18,4 | 61,7 | 59,4 | 52,2 | 53,0 |

(continued)

| STUDY GROUP (GS) | | | | | | | | | | |
| --- | --- | --- | --- | --- | --- | --- | --- | --- | --- | --- |
| Code | QUO | QU1 | PT0 | PT1 | FE0 | FE1 | CR0 | CR1 | CM0 | CM1 |
| FS.04 | 21,4 | 20,0 | 11,0 | 9,0 | 9,9 | 9,4 | 56,6 | 56,8 | 49,4 | 49,4 |
| FS.05 | 32,0 | 28,4 | 14,2 | 13,0 | 21,2 | 18,5 | 58,0 | 58,0 | 52,0 | 51,5 |
| FS.06 | 20,0 | 20,0 | 18,5 | 15,3 | 15,0 | 15,0 | 55,5 | 55,5 | 54,0 | 54,0 |
| FS.07 | 17,6 | 16,8 | 11,0 | 8,6 | 14,0 | 12,4 | 52,5 | 52,0 | 46,0 | 48,0 |
| FS.08 | 16,6 | 14,4 | 16,6 | 13,5 | 19,0 | 14,5 | 52,7 | 53,0 | 49,8 | 46,0 |
| FS.09 | 23,2 | 21,8 | 6,0 | 4,8 | 11,0 | 8,0 | 53,6 | 53,0 | 49,4 | 48,7 |
| FS.10 | 9,2 | 8,5 | 9,5 | 8,6 | 8,2 | 7,0 | 51,8 | 51,0 | 47,0 | 47,5 |
| FS.11 | 48,8 | 40,2 | 28,4 | 22,6 | 40,0 | 35,0 | 71,6 | 66,5 | 61,0 | 57,5 |
| FS.12 | 15,6 | 15,0 | 11,8 | 11,0 | 11,0 | 10,2 | 53,0 | 52,4 | 47,8 | 47,4 |
| FS.13 | 41,8 | 38,0 | 31,0 | 27,2 | 35,0 | 31,5 | 66,0 | 60,0 | 62,0 | 56,0 |
| FS.14 | 23,0 | 20,1 | 14,4 | 12,8 | 18,4 | 15,5 | 53,0 | 51,0 | 46,7 | 47,0 |
| FS.15 | 40,0 | 32,0 | 25,0 | 20,2 | 36,0 | 30,2 | 65,0 | 61,0 | 56,5 | 56,0 |

EXAMPLE 8

Lotion to prevent hair loss and to increase hair growth

| | % W/W |
| --- | --- |
| 01-DENATURATED ALCOHOL | 44.00 |
| 02-TIOCTIC ACID | 2.50 |
| 03-CYSTEINE, | 2.5 |
| 04-LECITHIN, | 2.00 |
| 05-PERFUME, | 0.20 |
| 06-TOCOPHERYL NICOTINATE, | 0.10 |
| 07-TOCOPHERYL LINOLEATE | 0.05 |
| 08-CYCLOMETHICONE, | 0.05 |
| 09-WATER | 45.65 |

(continued)

| | % W/W |
|---|---|
| 10-SODIUM HYDROXIDE, | 0.12 |
| 11-PEMULEN TR2 (Biochim) | 0.20 |
| 12-SODIUM STEAROYLGLUTAMATE, | 0.50 |

Solubilise in alcohol by stirring the above components 2, 3, 4, 5, 6, 7, 8 (A); solubilise sodium hydroxide in 10% of water (B); disperse in water at 60°C Pemulen TR2 (acrylates/$C_{10}$-$C_{30}$ alkyl acrylate crosspolymer) (C), then add sodium stearoylglutamate.

Add B to C and cool until 30°C, then add (A) by stirring. Other two lotions were prepared replacing cysteine with cystine and carboxymethylcystine.

Ten volunteers, 25 to 40 years old, were invited to use the lotion two times a day for two months with a suitable massage.

All volunteers had a beginning of alopecia with a heavy daily hair loss. Five volunteers were also affected by seborrhoeic dermatitis.

After the treatment all volunteers were controlled recording the subjective results. All patients showed an improvement in the look of the hair which appeared energised, shining and easy to comb. The alopecia area did not increase as well as the thinning out.

The volunteers were invited to control the daily hair loss: in the morning they had to brush for 10 minutes and control the hair loss. They had to evaluate their less according to the following scale:

1 very small loss, 2 small loss, 3 high loss, 4 very high loss.

During the first 15 days all volunteers had very high loss and at the end of the test reported that a reduced or very reduced number of hair could be found on their brushes. Also the seborrhoeic dermatitis resulted to be significantly reduced.

## Claims

1. A composition for the treatment of a pathological condition deriving from oxidative stresses, comprising tioctic acid and at least an antioxidant selected from the group consisting of cysteine, N-acetyl cysteine, cystine and carboxymethyl cysteine, **characterized in that** the weight ratio of the tioctic acid over said antioxidant in the composition is of 1:1 w/w.

## Patentansprüche

1. Zusammensetzung zur Behandlung eines pathologischen Zustands, der von oxidativen Stressen herrührt, umfassend Thioctsäure und wenigstens ein Antioxidans, ausgewählt aus der Gruppe bestehend aus Cystein, N-Acetylcystein, Cystin und Carboxymethylcystein, **dadurch gekennzeichnet, dass** das Gewichtsverhältnis der Thioctsäure gegenüber dem Antioxidans in der Zusammensetzung 1:1 Gew./Gew. ist.

## Revendications

1. Une composition pour le traitement d'un état pathologique dû à un stress oxydant, comprenant de l'acide alpha-lipoïque et au moins un antioxydant choisi parmi un groupe comprenant de la cystéine, de la N-acétyl cystéine, de la cystine et de la carboxyméthyl cystéine, **caractérisé en ce que** le taux massique de l'acide alpha-lipoique sur ledit antioxydant dans la composition est de 1:1 m/m